# EUROPEAN PATENT SPECIFICATION

(11) **EP 3 613 391 B1**
(45) Date of publication and mention of the grant of the patent: **31.08.2022**
(21) Application number: 17906552.9
(22) Date of filing: 21.04.2017
(51) Int. Cl.: A61F 13/15, A61F 13/49

(54) **METHOD OF MANUFACTURING ABSORBENT ARTICLE**
VERFAHREN ZUR HERSTELLUNG EINES SAUGFÄHIGEN ARTIKELS
PROCÉDÉ DE FABRICATION D'UN ARTICLE ABSORBANT

(43) Date of publication of application: 26.02.2020
(73) Proprietor: Unicharm Corporation, Shikokuchuo-shi, Ehime 799-0111 (JP)
(72) Inventor: TAKEUCHI, Kenji, Kanonji-shi Kagawa 769-1602 (JP); KAWAZU, Fumihito, Kanonji-shi Kagawa 769-1602 (JP)
(74) Representative: Staeger & Sperling Partnerschaftsgesellschaft mbB
(86) International application number: PCT/JP2017/016106
(87) International publication number: WO 2018/193634

(56) References cited:
- JP-A- 2008 012 005
- JP-A- 2009 072 532
- JP-A- 2014 104 261
- JP-A- 2014 171 690
- JP-U- H0 432 718

## Description

### TECHNICAL FIELD

The present invention relates to an absorbent article such as a disposable diaper.

### BACKGROUND ART

Patent Literature 1 discloses an absorbent article and a manufacturing method thereof. The absorbent article described in Patent Literature 1 includes an annular elastic waist panel extending in the direction around the waist of the wearer, an elastic crotch panel attached to the skin facing surface side of the elastic waist panel, and a base layer included in the crotch panel and having an absorbent structure in the central portion of the skin facing surface. The absorbent structure is fixed to the elastic waist panel by a hot melt adhesive.

### CITATION LIST

### PATENT LITERATURE

Patent Literature 1: JP 2014-171690 A. Further prior art in this technical field is disclosed in documents JP 2009 072532 A and JP 2008 012005 A.

### SUMMARY OF INVENTION

In the absorbent article described in Patent Literature 1, for example, when worn, the waist panel of the absorbent article is lifted upward. Moreover, when an absorbent structure absorbs a lot of wearer's bodily fluid, an absorbent structure tends to fall below due to the weight of a bodily fluid. Therefore, a strong load may be applied to the bonded portion between the absorbent structure and the waist panel. Therefore, it is desired that the bonding strength of the bonded portion between the absorbent structure and the waist panel be high.

A large amount of adhesive may be applied in order to increase the bonding strength of the bonded portion between the absorbent structure and the waist panel. However, if a large amount of adhesive is applied, the adhesive may ooze out from a sheet constituting the absorbent article, for example, a nonwoven fabric, or the touch of the sheet may deteriorate.

Therefore, it is desired to increase the bonding strength of the bonded portion between the absorbent structure and the waist panel without excessively increasing the amount of adhesive to be applied.

A method of manufacturing an absorbent article according to an the invention is defined in claim 1 and is a method of manufacturing an absorbent article including a waistline region, a crotch region, and an absorber disposed at least in the crotch region. The method of manufacturing comprises: a shaping step of forming a convexo-concave portion on a first sheet composed of a nonwoven fabric disposed in the crotch region; an absorber disposing step of disposing the absorber on the first sheet; and an adhesion step of bonding the first sheet to a waistline sheet disposed in the waistline region with a first adhesive after the shaping step. The adhesion step includes applying at least part of the first adhesive to a region which overlaps with the convexo-concave portion and does not overlap with the absorber in at least one of the first sheet and the waistline sheet.

### BRIEF DESCRIPTION OF DRAWINGS

Fig. 1 is a perspective view of an absorbent article according to an embodiment.
Fig. 2 is a developed plan view of an absorbent article according to an embodiment.
Fig. 3 is an exploded perspective view of an absorbent article according to an embodiment.
Fig. 4 is an exploded perspective view of part of an absorbent main body according to an embodiment.
Fig. 5 is a schematic cross sectional view taken along line V-V in Fig. 2.
Fig. 6 is a schematic view showing a method of manufacturing an absorbent article.
Fig. 7 is a schematic view showing a step that follows Fig. 6.
Fig. 8 is a diagram showing an example of a shaping roll for forming a shaping.
Fig. 9 is a diagram showing another example of a shaping roll for forming a shaping.

### Mode for Carrying Out the Invention

### (1) Outline of embodiment

At least the following matters will become apparent from the description of the present specification and the accompanying drawings.

A method of manufacturing an absorbent article according to an aspect is a method of manufacturing an absorbent article including a waistline region, a crotch region, and an absorber disposed at least in the crotch region. The method of manufacturing comprises: a shaping step of forming a convexo-concave portion on a first sheet composed of a nonwoven fabric disposed in the crotch region; an absorber disposing step of disposing the absorber on the first sheet; and an adhesion step of bonding the first sheet to a waistline sheet disposed in the waistline region with a first adhesive after the shaping step. The adhesion step includes applying at least part of the first adhesive to a region which overlaps with the convexo-concave portion and does not overlap with the absorber in at least one of the first sheet and the waistline sheet.

According to this aspect, at least part of a first adhesive is applied to a region that overlaps the convexo-concave portion in at least one of a first sheet and a waistline sheet. That is, the first sheet and the waistline sheet are bonded with the first adhesive at least in the region where the convexo-concave portion is formed. In this case, since the waistline sheet is bonded in a state of biting into the convexo-concave portion of the first sheet, the bonding strength between the first sheet disposed in the crotch region and the waistline sheet disposed in the waistline region can be further increased without excessive application amount of the adhesive.

According to a preferred aspect, the shaping step includes forming the convexo-concave portion extending along a direction intersecting with an orientation direction of fibers of the nonwoven fabric.

Generally, since it is easy to bend flexibly in the orientation direction of fibers of the nonwoven fabric as a reference point, it is difficult to put a firm crease (a fold) when folded in the orientation direction of fibers of the nonwoven fabric as a reference point. On the other hand, when the nonwoven fabric is folded in the direction intersecting the orientation direction of fibers of the nonwoven fabric as a reference point, it easy to put a firm crease (a fold).

According to this aspect, since the convexo-concave portion extending along the direction intersecting the orientation direction of fibers of the nonwoven fabric is formed, the convexo-concave portion can be formed firmly. As a result, the waistline sheet can be bonded in a state of firmly biting into the convexo-concave portion of the first sheet. According to a preferred aspect, the nonwoven fabric has a first surface and a second surface having a fiber density higher than a fiber density at the first surface, and wherein the adhesion step includes bonding the second surface of the nonwoven fabric to the waistline sheet.

The surface of the nonwoven fabric with a higher fiber density (second surface) has more fibers coming into contact with the waistline sheet than the surface of the nonwoven fabric with a lower fiber density (first surface). Therefore, by bonding the second surface of the nonwoven fabric to the waistline sheet, it is possible to further increase the bonding strength between the first sheet disposed in the crotch region and the waistline sheet disposed in the waistline region.

In addition, since the convexo-concave portion of the surface of the nonwoven fabric with a higher fiber density (second surface) is likely to be more firmly bonded than that of the surface of the nonwoven fabric with a lower fiber density (first surface), it is easy to bond the waistline sheet in a state where it firmly bites into the convexo-concave portion of the first sheet. Further, since the first surface of the nonwoven fabric has a low fiber density, the original softness of the nonwoven fabric can be maintained. That is, while increasing the bonding strength between the waistline sheet and the first sheet using the convexo-concave portion firmly attached to the surface of the nonwoven fabric with a higher fiber density (second surface), the original softness of the nonwoven fabric can be maintained because the second surface of the nonwoven fabric has a lower fiber density.

According to a preferred aspect, the waistline sheet is composed of a nonwoven fabric, the nonwoven fabric constituting the waistline sheet has a third surface and a fourth surface having a fiber density higher than a fiber density at the third surface, and the adhesion step includes bonding the first sheet to the fourth surface of the nonwoven fabric constituting the waistline sheet.

The surface of the nonwoven fabric with a higher fiber density (fourth surface) constituting the waistline sheet has more fibers coming into contact with the first sheet than the surface of the nonwoven fabric with a lower fiber density (third surface). Therefore, by bonding the first sheet to the fourth surface of the nonwoven fabric constituting the waistline sheet, the bonding strength between the first sheet disposed in the crotch region and the waistline sheet disposed in the waistline region can be further increased. Furthermore, the softness of the nonwoven fabric can be maintained by reducing the fiber density at the third surface of the nonwoven fabric. In other words, while increasing the bonding strength by firmly attaching to the first sheet the surface of the nonwoven fabric with a higher fiber density (fourth surface) that constitutes the waistline sheet, the fiber density at the third surface of the nonwoven fabric is low, so that the softness of the original nonwoven fabric can be maintained. According to a preferred aspect, the shaping step includes forming the convexo-concave portion in both end regions of the first sheet, and the adhesion step includes applying at least part of the first adhesive to the both end regions of the first sheet, the convexo-concave portion being formed in the both end regions, in at least one of the first sheet and the waistline sheet.

According to this aspect, the convexo-concave portions are provided in both end regions of the first sheet where the bonding strength is most desired to be increased. Therefore, since the waistline sheet is firmly bonded at both end regions of the first sheet (regions where the convexo-concave portion is formed), the bonding strength between the first sheet disposed in the crotch region and the waistline sheet disposed in the waistline region can be further increased.

According to a preferred aspect, the method of manufacturing an absorbent article comprises: a bonding step of bonding a second sheet to the first sheet, wherein at least one of the first sheet and the second sheet has a figure, a picture, or a color, and the shaping step includes forming the convexo-concave portion in a region that does not overlap with the figure, the picture, or the color.

Thereby, the problem that a figure, a picture, or a color is difficult to see under the influence of the convexo-concave portion can be suppressed. On the other hand, as described above, the bonding strength between the first sheet and the waistline sheet can be increased by using the convexo-concave portion. That is, by disposing the convexo-concave portion in a region that does not overlap with the figure, the picture, or the color, it is possible to improve the bonding strength using the convexo-concave portion without reducing the visibility of the figure, the picture, or the color. According to a preferred aspect, the shaping step includes forming the convexo-concave portion, while conveying the first sheet in a conveyance direction, using a roll having a plurality of protrusions extending in a direction intersecting the conveyance direction. When the first sheet is conveyed in the conveyance direction, the first sheet is pulled in the conveyance direction. The convexo-concave portion can be firmly formed on the first sheet by forming the convexo-concave portion on the first sheet by a roll having a plurality of protrusions extending in the direction intersecting the conveyance direction with being pulled in the conveyance direction.

As a result, in the adhesion step, since the waistline sheet is bonded in a state of biting into the convexo-concave portion of the first sheet, the bonding strength between the first sheet disposed in the crotch region and the waistline sheet disposed in the waistline region can be further increased.

According to a preferred aspect, the shaping step includes forming the convexo-concave portion, while conveying the first sheet in a conveyance direction, using a roll having a plurality of protrusions extending in the conveyance direction.

When the convexo-concave portion is formed using a roll having a plurality of protrusions extending in the conveyance direction, the length of the first sheet (nonwoven fabric) in the conveyance direction is substantially unchanged before and after the shaping process. Therefore, it is possible to suppress a conveyance failure due to a change in the length of the first sheet in the conveyance direction, and to convey the first sheet more accurately during the manufacturing process.

According to a preferred aspect, the adhesion step includes bonding the first sheet and the waistline sheet to each other after the first adhesive is applied to the waistline sheet.

When trying to apply the first adhesive to the first sheet, it is difficult to apply the first adhesive to the end of the first sheet. According to this aspect, since the adhesive is applied to the waistline sheet, it is possible to accurately apply the first adhesive to the entire bonded portion with the first sheet. Therefore, it is possible to further increase the bonding strength between the first sheet disposed in the crotch region and the waistline sheet disposed in the waistline region.

According to a preferred aspect, the method of manufacturing the absorbent article comprises a step of bonding an elastic member to a region of the first sheet, the convexo-concave portion being formed in the region, after the shaping step.

By bonding the elastic member after the shaping step, it is possible to prevent the elastic member from being cut by a roll having a plurality of protrusions during the shaping step, for example.

According to a preferred aspect, the shaping step includes forming the convexo-concave portion in an end region of the first sheet in an intersecting direction intersecting a conveyance direction, the method comprises a folding back step of folding back the first sheet at an imaginary fold line, as a reference point, passing through a region in which the convexo-concave portion of the first sheet is formed after the shaping step, and bonding the folded portion of the first sheet with a second adhesive, and the folding back step includes applying the second adhesive only to part of the folded portion.

If a second adhesive is applied to the entire surface of the folded portion, the folded portion is rigidly bonded, and the rigidity of the folded portion of the first sheet may be excessively increased.

According to this aspect, the second adhesive is applied only to part of the folded portion. Therefore, the folded portion of the first sheet is relatively soft. In general, the amount of adhesive necessary to bond the soft part of the sheet (nonwoven fabric) is small. Therefore, by applying the second adhesive only to part of the folded portion, even if the amount of the first adhesive is small, it is possible to secure the bonding strength between the waistline sheet and the first sheet in the later adhesion step.

### (2) Configuration of absorbent article

Hereinafter, an embodiment will be described with reference to the drawings. In the following description of the drawings, the same or similar parts are denoted by the same or similar reference symbols. However, note that the drawings are schematic, and ratios of dimensions may be different from actual ones. Therefore, specific dimensions and the like are determined in consideration of the following description. Moreover, there may be portions where dimensional relationships or proportions are different among the drawings.

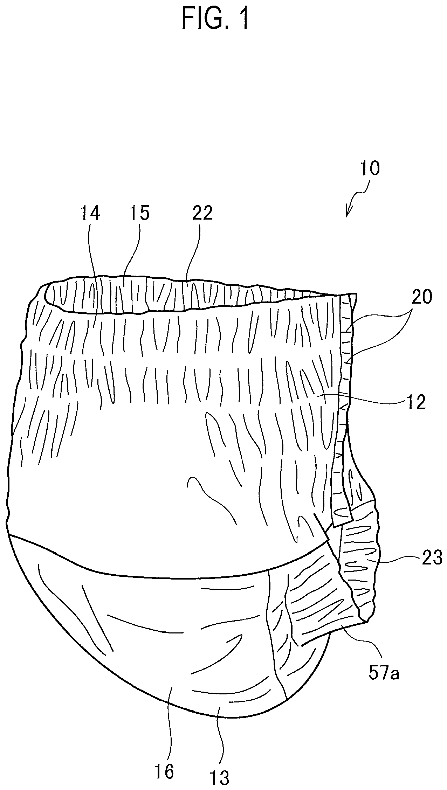

Fig. 1 is a perspective view of an absorbent article according to an embodiment. In the present embodiment, an absorbent article 10 is a so-called pants-type disposable diaper.

However, the absorbent article 10 of the present invention is not limited to a pants-type disposable diaper.

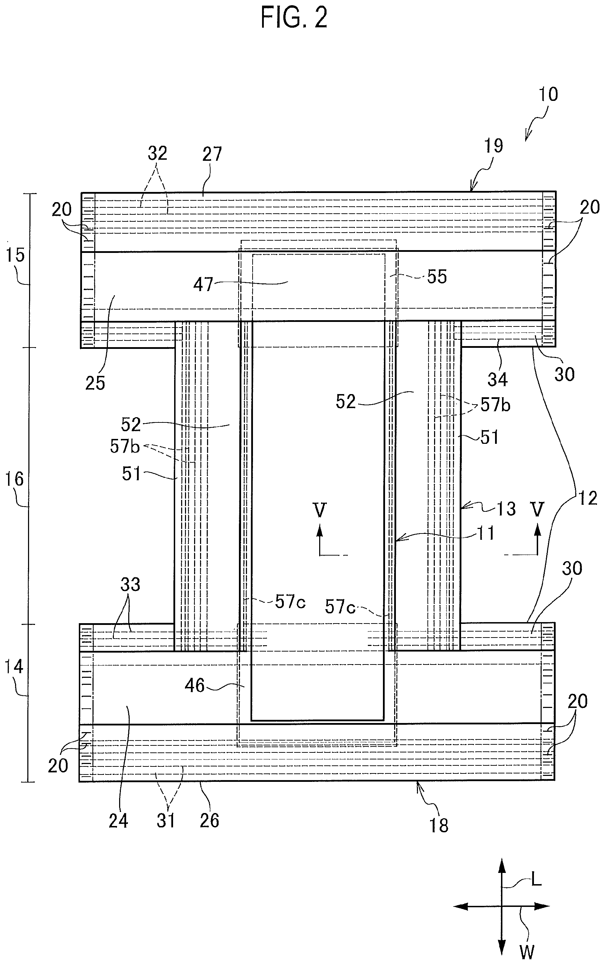

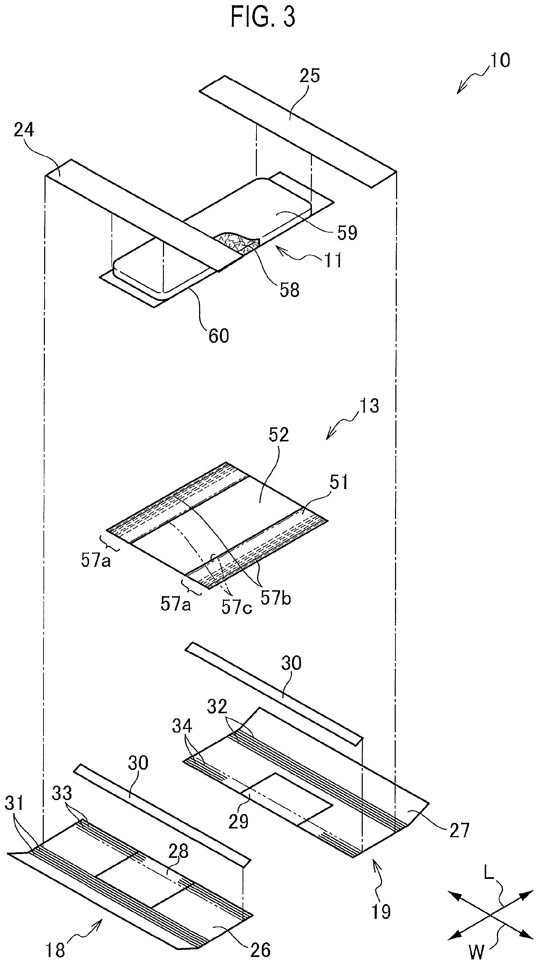

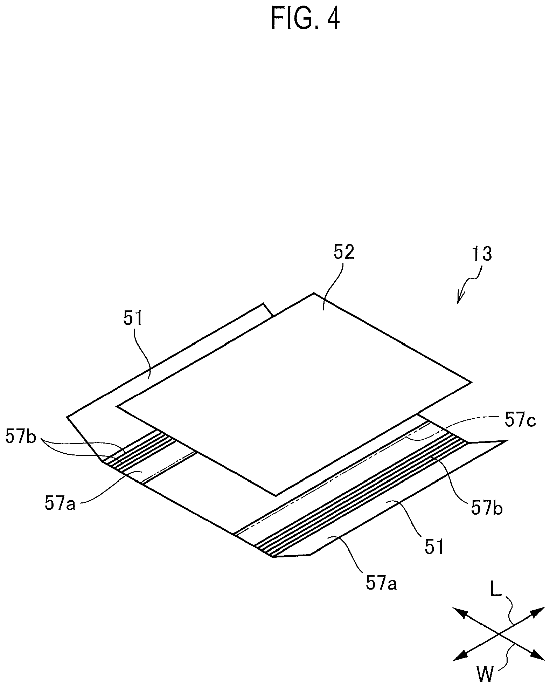

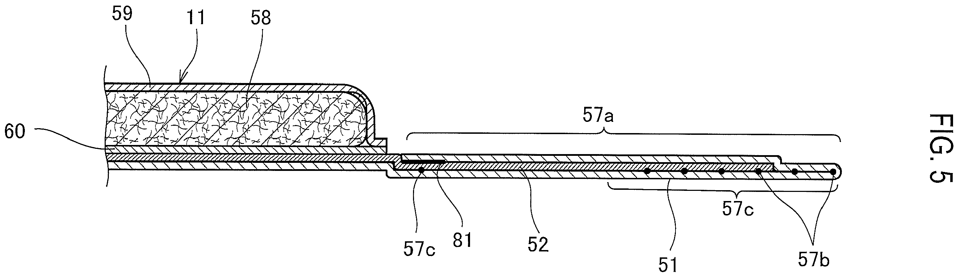

Fig. 2 is a developed plan view of an absorbent article according to an embodiment. Fig. 2 shows a plan view of a state in which a seam portion 20 described later is opened and an absorbent article 20 is developed. Fig. 3 is an exploded perspective view of components constituting the absorbent article 10. Fig. 4 is an exploded perspective view of part of an absorbent main body 13 constituting the absorbent article 10. Fig. 5 is a schematic cross-sectional view of the absorbent main body 13 taken along line V-V in Fig. 2.

The absorbent article 10 has waistline regions 14 and 15 disposed around the wearer's waist and a crotch region 16 disposed in the wearer's crotch. The waistline region includes a front waistline region 14 disposed around the wearer's front waist and a rear waistline region 15 disposed around the wearer's rear waist.

The absorbent article 10 has a front-back direction L that extends between the front waistline region 14 and the rear waistline region 15, and a width direction W that is orthogonal to the front-back direction L.

The absorbent article 10 includes a waist panel 12 constituting the waistline regions 14 and 15, and the absorbent main body 13 disposed at least in the crotch region 16. The waist panel 12 includes a front waistline sheet 18 disposed in the front waistline region 14 and a rear waistline sheet 19 disposed in the rear waistline region 15. The front waistline sheet 18 may have a rectangular shape extending in the width direction W. The rear waistline sheet 19 may have a rectangular shape extending in the width direction W.

The edge of the front waistline sheet 18 in the width direction W is bonded to the edge of the rear waistline sheet 19 in the width direction W at the seam portion 20. As a result, the waist panel 12 forms a waistline surrounding opening 22 into which the wearer's waist is inserted. Further, the absorbent article 10 according to the present embodiment has a leg surrounding opening 23 for inserting a wearer's leg.

Each of the front waistline sheet 18 and the rear waistline sheet 19 may include inner layer sheets 24 and 25 located on the skin surface and outer layer sheets 26 and 27 located on the non-skin surface. The outer layer sheets 26 and 27 may extend outward of the inner layer sheets 24 and 25 in the front-back direction L.

The outer layer sheets 26 and 27 may be composed of a nonwoven fabric. As this nonwoven fabric, for example, a spunbond nonwoven fabric or an air-through nonwoven fabric can be used. The inner layer sheets 24 and 25 arc bonded to the outer layer sheets 26 and 27 by an adhesive such as a hot melt adhesive.

The inner layer sheets 24 and 25 may be composed of a nonwoven fabric. As this nonwoven fabric, for example, a spunbond nonwoven fabric, a melt blown nonwoven fabric, an air-through nonwoven fabric, or the like can be used.

The front waistline sheet 18 may include elastic members 31 and 33 that are extensible in the width direction W. The elastic members 31 and 33 are bonded to the front waistline sheet 18 in an extended state, and contract the front waistline sheet 18 in the width direction W. The elastic member 31 may be provided in the vicinity of the front side end of the front waistline sheet 18 in the front-back direction L. The elastic member 33 may be provided in the vicinity of the rear side end of the front waistline sheet 18 in the front-back direction L.

The rear waistline sheet 19 may include elastic members 32 and 34 that are extensible in the width direction W. The elastic members 32 and 34 are bonded to the rear waistline sheet 19 in an extended state, and contract the rear waistline sheet 19 in the width direction W. The elastic member 32 may be provided in the vicinity of the rear side end of the rear waistline sheet 19 in the front-back direction L. The elastic member 34 may be provided in the vicinity of the front side end portion of the rear waistline sheet 19 in the front-back direction L.

Further, each of the elastic members 33 and 34 may be covered by an elongated fixing sheet 30 formed of, for example, nonwoven fabric.

The absorbent main body 13 extends from the front waistline sheet 18 to the rear waistline sheet 19. A front side end 46 of the absorbent main body 13 is bonded to the front waistline sheet 18 with an adhesive such as a hot melt adhesive. The rear side end 47 of the absorbent main body 13 is bonded to the rear waistline sheet 19 with an adhesive such as a hot melt adhesive.

The absorbent main body 13 includes an absorber 11 disposed at least in the crotch region 16. The absorber 11 includes an absorbent core 58, a top sheet 59 positioned on the skin surface of the absorbent core 58, and a back sheet 60 positioned on the non-skin surface of the absorbent core 58. The absorbent core 58 may include pulp, super absorbent polymer particles (SAP), and the like. The top sheet 59 may be made of a liquid-permeable nonwoven fabric. The back sheet 60 may be composed of a liquid-impermeable nonwoven fabric or a plastic sheet.

The absorbent main body 13 may include a crotch sheet 50 closer to the non-skin surface than the absorbent core 58. The crotch sheet 50 may include a first sheet 51 and a second sheet 52.

The first sheet 51 is composed of a nonwoven fabric. The first sheet 51 is provided at least in the crotch region 16 and extends from the front waistline region 14 to the rear waistline region 15. In the present embodiment, the first sheet 51 is a member that forms the leg surrounding opening 23 of a diaper 10.

The second sheet 52 is bonded to the first sheet 51. In the present embodiment, the second sheet 52 is bonded to the skin surface side of the first sheet 51. The second sheet 52 may be configured by a plastic sheet.

The first sheet 51 is bonded to the waistline sheet (the front waistline sheet 18 and the rear waistline sheet 19) of the waist panel 12 with a first adhesive such as a hot melt adhesive. Specifically, the first sheet 51 may be bonded to the outer layer sheets 26 and 27 of the front waistline sheet 18 and the rear waistline sheet 19. In this case, the inner layer sheets 24 and 25 of the front waistline sheet 18 and the rear waistline sheet 19 may be bonded to the skin surface side of the first sheet 51 and the second sheet 52. The first sheet 51 has a convexo-concave portion (Shaping) 57a. The convexo-concave portion 57a can be formed by passing at least the first sheet 51 between a pair of rolls having a plurality of protrusions.

The convexo-concave portion 57a may be formed in both end regions of the first sheet 51 in the width direction W, for example. In this case, the convexo-concave portion 57a may not be formed in the central region of the first sheet 51 in the width direction W. The region where the convexo-concave portion 57a is formed may extend from the front waistline region 14 to the rear waistline region 15.

The convexo-concave portion (shaping) 57a preferably extends along a direction intersecting the orientation direction of fibers of the nonwoven fabric constituting the first sheet 51. That is, it is preferable that each of the recesses and the projections constituting the convexo-concave portion 57a extend along the direction intersecting the orientation direction of fibers of the nonwoven fabric.

The front waistline sheet 18 and the first sheet 51, and the rear waistline sheet 19 and the first sheet 51 are bonded to each other by a first adhesive 28, 29 such as a hot melt adhesive. At least part of the first adhesive 28, 29 is applied to a region that overlaps with the convexo-concave portion 57a and does not overlap with the absorber 11 in at least one of the first sheet 51, and the front waistline sheet 18 and the rear waistline sheet 19. Here, part of the first adhesive 28, 29 may be applied to a region overlapping with the absorber 11.

Preferably, the first adhesive 28, 29 is applied to the both end regions of the first sheet 51 in the width direction W and the regions overlapping with the convexo-concave portion 57a. More preferably, the first adhesive is applied to the entire region where the front waistline sheet 18 and the first sheet 51, and the rear waistline sheet 19 and the first sheet 51 overlap and in which the convexo-concave portion 57a is formed. The nonwoven fabric constituting the first sheet 51 may have a first surface and a second surface having a fiber density higher than the fiber density at the first surface. In this case, it is preferable that the second surface of the nonwoven fabric constituting the first sheet 51 be bonded to the front waistline sheet 18 and the rear waistline sheet 19.

Further, the front waistline sheet 18 and the rear waistline sheet 19 may be composed of a nonwoven fabric. In this case, the nonwoven fabric constituting the front waistline sheet 18 and the rear waistline sheet 19 may have a third surface and a fourth surface having a fiber density higher than the fiber density at the third surface. In this case, it is preferable that the first sheet 51 be bonded to the fourth surface of the nonwoven fabric constituting the front waistline sheet 18 and the rear waistline sheet 19.

The first sheet 51 has an elastic member in a region where the convexo-concave portion 57a is formed. This elastic member may include a leg surrounding elastic member 57b and an additional elastic member 57c. The leg surrounding elastic member 57b and the additional elastic member 57c may be bonded to the first sheet 51 in an extended state.

At least one of the leg surrounding elastic member 57b and the additional elastic member 57c, that is, in this embodiment, the leg surrounding elastic member 57b, is preferably provided in a region where the convexo-concave portion 57a of the first sheet 51 is formed.

The first sheet 51 is folded back with an imaginary fold line, as a reference point, passing through the region where the convexo-concave portion 57a is formed. The folded portion of the first sheet 51 is bonded so as not to be developed by a second adhesive such as a hot melt adhesive. However, the second adhesive is preferably applied only to part of the folded portion of the first sheet 51.

At least one of the first sheet 51 and the second sheet 52 may have the figure, the picture, or the color. In this case, the convexo-concave portion 57a is preferably formed in a region that does not overlap with the figure, the picture, or the color. Specifically, the figure, the picture, or the color is preferably arranged in the central region of the first sheet 51 or the second sheet 52 in the width direction W.

In addition, part of the figure, the picture, or the color provided on at least one of the first sheet 51 and the second sheet 52 may be provided in a region overlapping with the convexo-concave portion 57a. For example, less than 5% of an area of the figure, the picture, or the color area may be provided in an area overlapping with the convexo-concave portion 57a.

### (3) Method of manufacturing absorbent article

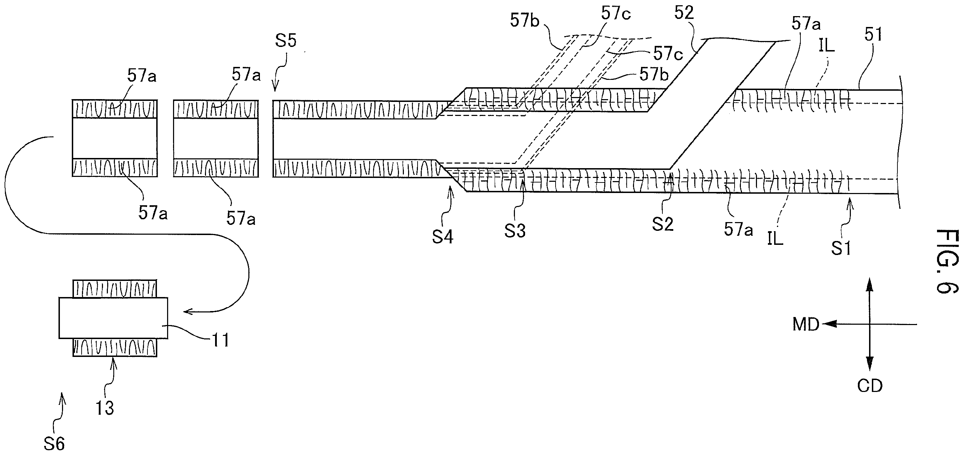

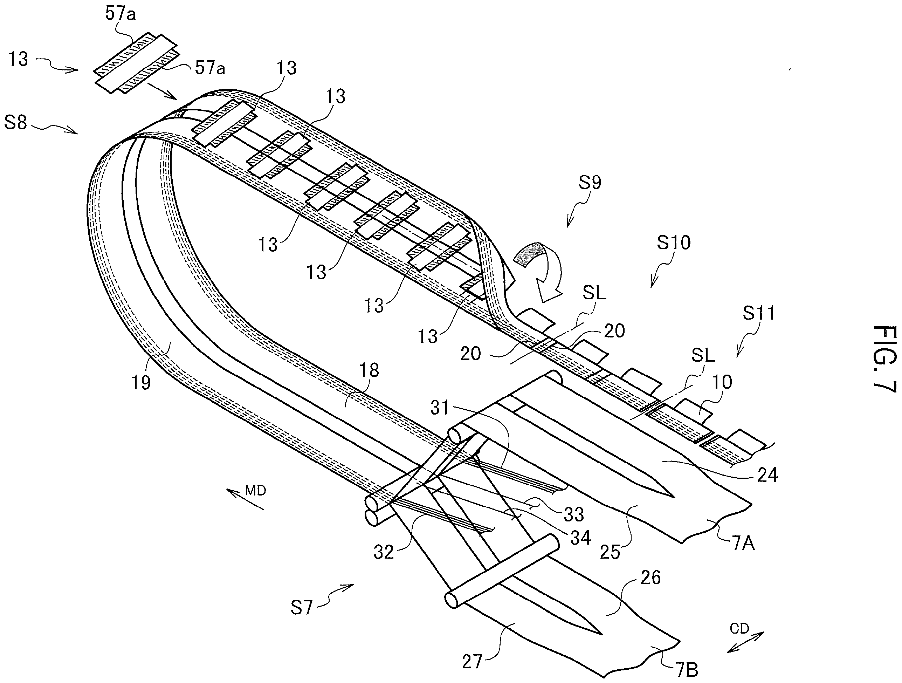

A method of manufacturing an absorbent article will be described with reference to Figs. 6 and 7. Figs. 6 and 7 are schematic views showing a method of manufacturing an absorbent article. Fig. 7 shows a step following Fig. 6. Reference symbol "MD" in the drawing corresponds to the machine direction, that is, the conveyance direction of the sheets constituting the absorbent article. The reference symbol "CD" in the drawing corresponds to the crossing direction intersecting the machine direction MD.

The method of manufacturing the absorbent article 10 may include a shaping step S1, a bonding step S2, an elastic member installation step S3, a folding back step S4, a first cutting step S5, an absorber disposing step S6, a waistline sheet forming step S7, an adhesion step S8, a folding step S9, a seam portion forming step S10, and a second cutting step S11. These steps S1 to S11 are preferably performed in this order. However, it should be noted that the order of these steps S1 to S11 can be interchanged as much as possible.

### <Shaping step S1>

In the method of manufacturing an absorbent article according to this embodiment, first, a line for manufacturing the absorbent main body 13 will be described. First, in the shaping step S1, the convexo-concave portion 57a is formed on the first sheet 51 composed of a nonwoven fabric disposed in the crotch region 16. The first sheet 51 constitutes a sheet continuous in the machine direction MD.

In the present embodiment, the convexo-concave portion 57a is formed in both end regions of the first sheet 51 in the cross direction CD. The region where the convexo-concave portion 57a is formed may extend long along the machine direction MD.

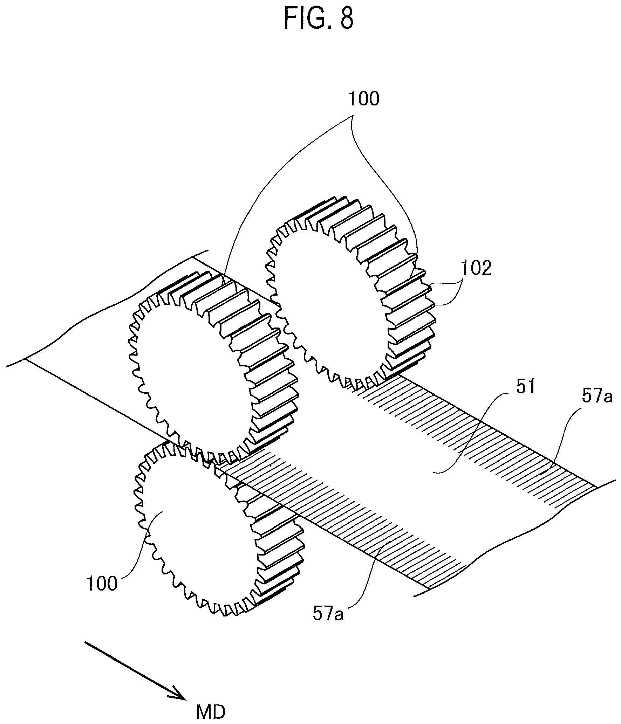

The convexo-concave portion 57a can be formed by using a shaping roll while conveying the first sheet 51 in the conveyance direction. Fig. 8 is a diagram illustrating an example of a shaping roll for forming a shaping. A shaping roll 100 shown in Fig. 8 has a plurality of protrusions 102 extending in the cross direction CD intersecting the conveyance direction MD of the first sheet 51.

When the first sheet 51 is conveyed in the conveyance direction MD, the first sheet 51 is pulled in the conveyance direction MD. By forming the convexo-concave portion 57a on the first sheet 51 by the roll 100 having the plurality of protrusions 102 extending in the direction CD intersecting the conveyance direction MD with being pulled in the conveyance direction MD, the convexo-concave portion 57a can be firmly formed on the first sheet 51. As a result, in the adhesion step S8 described later, the waistline sheets (the front waistline sheet 18 and the rear waistline sheet 19) are bonded in a state of further biting into the convexo-concave portion 57a of the first sheet 51 as described later. Therefore, it is possible to further increase the bonding strength between the first sheet 51 disposed in the crotch region 16 and the waistline sheet (the front waistline sheet 18 and the rear waistline sheet 19). In the following, the front waistline sheet 18 and the rear waistline sheet 19 may be collectively simply referred to as "a waistline sheet".

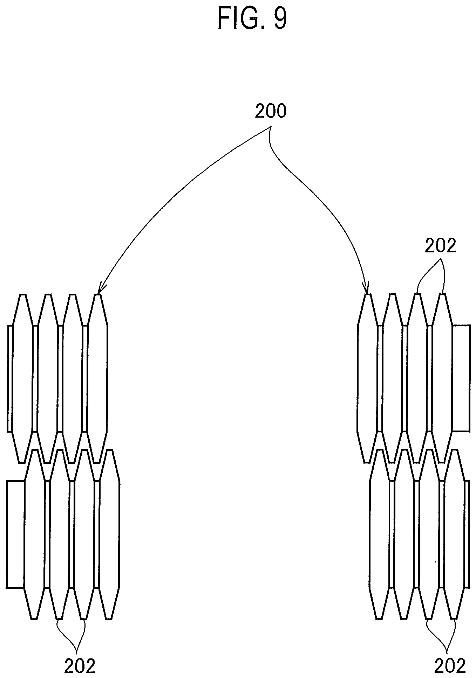

Fig. 9 is a diagram showing another example of the shaping roll for forming the shaping. The shaping roll 200 shown in Fig. 9 has a plurality of protrusions 202 extending in the conveyance direction MD. When the convexo-concave portion 57a is formed using the roll 200 having the plurality of protrusions 202 extending in the conveyance direction MD, the length of the first sheet (nonwoven fabric) 51 in the conveyance direction MD is substantially unchanged before and after the shaping step S1. Therefore, it is possible to suppress a conveyance failure due to a change in the length of the first sheet 51 in the conveyance direction MD, and to convey the first sheet 51 more accurately during the manufacturing process.

As shown in Figs. 8 and 9, the convexo-concave portion 57a are formed in both end regions of the first sheet 51 in the cross direction CD. That is, the convexo-concave portion 57a may not be formed in the central region of the first sheet 51 in the cross direction CD. In this case, the shaping roll may include two pairs of rolls 100 and 200 that are spaced apart in the cross direction CD.

Preferably, in the shaping step S1, the convexo-concave portion 57a extending along the direction intersecting the orientation direction of fibers of the nonwoven fabric constituting the first sheet 51 is formed. Generally, since the nonwoven fabric is easy to bend flexibly in the orientation direction of fibers of the nonwoven fabric as a reference point, it is difficult to put a firm crease (a fold) when folded in the orientation direction of fibers of the nonwoven fabric as a reference point. On the other hand, when the nonwoven fabric is folded in the direction intersecting the orientation direction of fibers of the nonwoven fabric as a reference point, it easy to put a firm crease (a fold).

According to this aspect, since the convexo-concave portion 57a extending along the direction intersecting the orientation direction of fibers of the nonwoven fabric is formed, the convexo-concave portion 57a can be firmly formed. Accordingly, the waistline sheet 18, 19 can be bonded in a state of firmly biting into the convexo-concave portion 57a of the first sheet 51 in the adhesion step S8 described later.

### <Bonding step S2>

In the bonding step S2, the second sheet 52 is bonded to the first sheet 51 conveyed on the conveyance line. The second sheet 52 may preferably be configured by a plastic sheet. Note that the bonding step S2 may be performed as necessary.

In the cross direction CD, the width of the second sheet 52 may be narrower than the width of the first sheet 51. In this case, it is preferable that at least part of the convexo-concave portion 57a formed on the first sheet 51 be exposed from the second sheet 52.

Here, at least one of the first sheet 51 and the second sheet 52 may have the figure, the picture, or the color. In this case, preferably, in the shaping step S1, the convexo-concave portion 57a is formed in a region that does not overlap with the figure, the picture, or the color. Thereby, when the absorbent article 10 is completed, it is possible to prevent the figure, the picture, or the color from being difficult to see due to the influence of the convexo-concave portion 57a. On the other hand, as will be described later, it is possible to increase the bonding strength between the first sheet 51 and the waistline sheet 18, 19 by using the convexo-concave portion 57a. That is, by disposing the convexo-concave portion 57a in a region that does not overlap with the figure, the picture, or the color, it is possible to improve the bonding strength using the convexo-concave portion 57a without reducing the visibility of the figure, the picture, or the color.

In this embodiment, the convexo-concave portion 57a may be formed on both sides of the first sheet 51 in the cross direction CD, and the figure, the picture, or the color may be provided in the central region of the second sheet 52 in the cross direction CD.

In addition, part of the figure, the picture, or the color provided on at least one of the first sheet 51 and the second sheet 52 may be provided in a region overlapping with the convexo-concave portion 57a. For example, less than 5% of an area of the figure, the picture, or the color area may be provided in an area overlapping with the convexo-concave portion 57a.

Preferably, the second sheet 52 is made of a plastic sheet, and the figure, the picture, or the color is formed on the second sheet 52. Since the second sheet 52 is made of a plastic sheet, the figure, the picture, or the color can be easily formed by using, for example, an ink layer.

Further, when the second sheet 52 composed of a plastic sheet is bonded to the first sheet 51 composed of a nonwoven fabric, the bonding step S2 is preferably performed after the shaping step S1. This is because the plastic sheet is prevented from being broken by the shaping rolls 100 and 200.

### <Elastic member installation step S3>

After the shaping step SI, the clastic member installation step S3 for bonding the elastic member 57b may be performed in the region where the convexo-concave portion 57a of the first sheet 51 is formed. In the present embodiment, as described above, the elastic member 57b is a leg surrounding elastic member. Further, in the elastic member installation step S3, not only the leg surrounding elastic member 57b but also the additional elastic member 57c may be installed on the first sheet 51.

The leg surrounding elastic member 57b and/or the additional elastic member 57c are bonded to the first sheet 51 in an extended state by, for example, a hot melt adhesive.

The leg surrounding elastic member 57b and the additional elastic member 57c can cause the absorbent article 10 to easily fit around the legs of the user, and can provide an enhanced comfortable absorbent article. By bonding such an elastic member 57b after the shaping step S1, it is possible to prevent the elastic member 57b from being cut by the rolls 100 and 200 during the shaping step S1, for example.

### <Folding back step S4>

The folding back step S4 may be performed in which the first sheet 51 is folded back at the imaginary fold line IL, as a reference point, passing through the region where the convexo-concave portion 57a of the first sheet 51 is formed after the shaping step S1, and the folded portion of the first sheet 51 is bonded with an adhesive. By folding back both end regions of the first sheet 51 in the cross direction CD, the leg surrounding elastic member 57b and the additional elastic member 57c can be disposed inside the first sheet 51 (see also Fig. 5).

In addition, when the second sheet 52 is bonded on the first sheet 51, it is preferable that the portion of the first sheet 51 that extends outward of the second sheet 52 in the cross direction CD be folded back. In this case, the folded portion of the first sheet 51 may cover the end region of the second sheet 52 in the cross direction CD. That is, the end region of the second sheet 52 in the cross direction CD may be sandwiched between the first sheet 51 in the thickness direction.

Further, the first sheet 51 may be made to pass between a pair of nip rolls (not shown) in order to securely bond the inner surfaces of the folded first sheet 51.

In the folding back step S4, the adhesive that bonds the folded portion of the first sheet 51 so that it cannot be unfolded is preferably applied only to part of the folded portion. That is, if an adhesive is applied to the entire surface of the folded portion of the first sheet 51, the folded portion is rigidly bonded, and the rigidity of the folded portion of the first sheet 51 may become excessively high.

According to this aspect, the adhesive is applied only to part of the folded portion, so that the folded portion of the first sheet 51 is relatively soft. In general, the amount of adhesive necessary to bond the soft part of the sheet (nonwoven fabric) is small. Therefore, by applying the adhesive to only part of the folded portion, even if the amount of the adhesive used in the subsequent adhesion step is small, the bonding strength between the waistline sheet and the first sheet 51 can be ensured.

### <First cutting step S5>

In the first cutting step S5, the continuously connected first sheet 51 is cut into a size used for one product. Specifically, the first sheet 51 can be cut by causing the first sheet 51 to pass between a cutter and an anvil roller that are arranged to face each other.

### <Absorber disposing step S6>

In the absorber disposing step S6, the absorber 11 is arranged on the first sheet 51. As described above, the absorber 11 may include the absorbent core 58, the top sheet 59, and the back sheet 60. In addition, it is preferable that at least part of the convexo-concave portion 57a formed on the first sheet 51 in the shaping step S1 be disposed in a region that does not overlap with the absorbent core 58. The absorbent main body 13 is formed through the absorber disposing step S6.

### <Waistline sheet forming step S7>

Next, the absorbent main body 13 is mounted on the waistline sheet (the front waistline sheet 18 and the rear waistline sheet 19). First, in the waistline sheet forming step S7, while conveying webs 7A and 7B constituting the front waistline sheet 18 and the rear waistline sheet 19, a cut is made in the center of the webs 7A and 7B, and each of the webs 7A and 7B is divided into two regions in the cross direction CD. As a result, as shown in Fig. 7, the inner layer sheets 24 and 25 of the front waistline sheet 18 and the rear waistline sheet 19, and the outer layer sheets 26 and 27 of the front waistline sheet 18 and the rear waistline sheet 19 are formed.

Then, the aforementioned elastic members 31, 32, 33, and 34 are sandwiched between the inner layer sheets 24 and 25 and the outer layer sheets 26 and 27. Thus, the front waistline sheet 18 and the rear waistline sheet 19 are formed. The front waistline sheet 18 and the rear waistline sheet 19 are moved side by side with a space in the cross direction CD.

### <Adhesion step S8>

After the shaping step S1, the adhesion step S8 is performed in which the first sheet 51 is bonded to the front waistline sheet 18 and the rear waistline sheet 19 disposed in the front waistline region 14 and the rear waistline region 15, respectively, with the first adhesive 28, 29. In the present embodiment, in the adhesion step S8, the absorbent main body 13 is disposed over the front waistline sheet 18 and the rear waistline sheet 19.

In the adhesion step S8, at least part of the first adhesive 28, 29 is applied to a region that overlaps with the convexo-concave portion 57a and does not overlap with the absorber 11 in at least one of the first sheet 51 and the waistline sheet 18, 19 (see also Fig. 3). Part of the first adhesive 28, 29 may be applied to a region overlapping with the absorber 11.

According to this aspect, at least part of the first adhesive 28, 29 is applied to a region overlapping the convexo-concave portion 57a in at least one of the first sheet 51 and the waistline sheet 18, 19. That is, the first sheet 51 and the waistline sheet 18, 19 are bonded by the first adhesive 28, 29 at least in the region where the convexo-concave portion 57a is formed. In this case, since the waistline sheet 18, 19 is bonded in a state of biting into the convexo-concave portion 57a of the first sheet 51, the bonding strength between the first sheet 51 disposed in the crotch region 16 and the waistline sheet 18, 19 can be further increased without excessive application amount of the first adhesive 28, 29.

Preferably, in the adhesion step S8, at least part of the first adhesive 28, 29 is applied to both end regions where the convexo-concave portion 57a of the first sheet 51 is formed in at least one of the first sheet 51 and the waistline sheet 18, 19. When the convexo-concave portion 57a is formed in both end regions, which is desired to have the highest bonding strength, of the first sheet 51, by applying the first adhesive 28, 29 to both end regions of the first sheet 51, the waistline sheet 18, 19 is firmly bonded to both end regions of the first sheet 51 (regions where the convexo-concave portion 57a is formed). Therefore, the bonding strength between the first sheet 51 disposed in the crotch region 16 and the waistline sheet 18, 19 disposed in the waistline region can be further increased.

Preferably, in the adhesion step S8, after the first adhesive 28, 29 are applied to the waistline sheet 18, 19, the first sheet 51 and the waistline sheet 18, 19 are bonded to each other. If the first adhesive 28, 29 are to be applied to the first sheet 51, it is difficult to apply the first adhesive 28, 29 to the edge of the first sheet 51. According to this aspect, since the first adhesive 28, 29 are applied to the waistline sheet 18, 19, it is possible to accurately apply the first adhesive 28, 29 to the entire bonded portion with the first sheet 51. Therefore, it is possible to further increase the bonding strength between the first sheet 51 disposed in the crotch region 16 and the waistline sheet 18, 19 disposed in the waistline region.

The nonwoven fabric constituting the first sheet 51 may have a first surface and a second surface having a fiber density higher than the fiber density at the first surface. In this case, in the adhesion step S8, it is preferable to bond the second surface of the nonwoven fabric constituting the first sheet 51 to the waistline sheet 18, 19. The surface of the nonwoven fabric with a higher fiber density (second surface) has more fibers coming into contact with the waistline sheet 18, 19 than the surface of the nonwoven fabric with a lower fiber density (first surface). Therefore, by bonding the second surface of the nonwoven fabric to the waistline sheet 18, 19, it is possible to further increase the bonding strength between the first sheet 51 disposed in the crotch region 16 and the waistline sheet 18, 19 disposed in the waistline region.

In addition, since the convexo-concave portion 57a of the surface of the nonwoven fabric with a higher fiber density (second surface) constituting the first sheet 51 is likely to be more firmly bonded than that of the surface of the nonwoven fabric with a lower fiber density (first surface), it is easy to bond the waistline sheet 18, 19 in a state where it firmly bites into the convexo-concave portion 57a of the first sheet 51.

Furthermore, the softness of the nonwoven fabric constituting the first sheet 51 can be maintained by reducing the fiber density at the first surface of the nonwoven fabric. That is, while increasing the bonding strength between the waistline sheet 18, 19 and the first sheet 51 using the convexo-concave portion 57a firmly attached to the surface of the nonwoven fabric with a higher fiber density (second surface), the original softness of the nonwoven fabric can be maintained because the second surface of the nonwoven fabric has a lower fiber density.

Further, the waistline sheet 18, 19 may be composed of a nonwoven fabric. The nonwoven fabric constituting the waistline sheet 18, 19 may have a third surface and a fourth surface having a fiber density higher than the fiber density at the third surface. In the adhesion step S8, the first sheet 51 is preferably bonded to the fourth surface of the nonwoven fabric constituting the waistline sheet 18, 19. The surface of the nonwoven fabric with a higher fiber density (fourth surface) constituting the waistline sheet 18, 19 has more fibers coming into contact with the first sheet 51 than the surface of the nonwoven fabric with a lower fiber density (third surface). Therefore, by bonding the first sheet 51 to the fourth surface of the nonwoven fabric constituting the waistline sheet 18, 19, it is possible to further increase the bonding strength between the first sheet 51 disposed in the crotch region 16 and the waistline sheet 18, 19 disposed in the waistline region.

Furthermore, the softness of the nonwoven fabric constituting the waistline sheet 18, 19 can be maintained by reducing the fiber density at the third surface of the nonwoven fabric. That is, while increasing the bonding strength by firmly bonding the surface of the nonwoven fabric with a higher fiber density (fourth surface) constituting the waistline sheet 18, 19 to the first sheet 51, the third surface of the nonwoven fabric has a lower fiber density, so that the original softness of the nonwoven fabric can be maintained.

In addition, it is preferable that the adhesion step S8 be performed without performing the step of bonding, to the first sheet 51, another sheet conveyed at a speed different from the conveyance speed of the first sheet 51 after the shaping step S1. If there is a step of bonding another sheet conveyed at a speed different from the conveyance speed of the first sheet 51 to the first sheet 51 between the shaping step S1 and the adhesion step S8, the two sheets are bonded in a state of different stretch rate, so that the convexo-concave portion 57a formed on the first sheet 51 may collapse. That is, the convexo-concave portion 57a of the first sheet 51 may become smoother. In this case, the advantage that the waistline sheet 18, 19 is bonded in a state of biting into the convexo-concave portion 57a of the first sheet 51 may be reduced.

According to this aspect, since the adhesion step S8 is performed without performing the step of bonding, to the first sheet 51, another sheet conveyed at a speed different from the conveyance speed of the first sheet 51 after the shaping step S1, the waistline sheet 18, 19 can be bonded to the first sheet 51 in a state of firmly biting into the convexo-concave portion 57a of the first sheet 51. However, if the convexo-concave portion 57 of the first sheet 51 is sufficiently maintained, the step of bonding another sheet conveyed at a speed different from the conveyance speed of the first sheet 51 to the first sheet 51 may be performed between the shaping step S1 and the adhesion step S8.

### <Folding step S9>

Next, the waistline sheet 18, 19 on which the absorbent main body 13 is installed is folded in two at a fold line along the conveyance direction MD as a reference point. As a result, the front waistline sheet 18 overlaps the rear waistline sheet 19.

### <Seam portion forming step S10>

Next, in the seam portion forming step S10, the seam portion 20 is formed by bonding the front waistline sheet 18 and the rear waistline sheet 19 to each other at a position corresponding to the outer end, in the width direction, of each of the absorbent articles 10 that are continuously connected. Thereby, the absorbent article 10 is formed in a pants shape.

### <Second cutting step S11>

Next, in the second cutting step S11, respective products are obtained by cutting the continuously connected absorbent article 10 at the seam portion 20.

As mentioned above, although the present invention is explained in detail using the above-mentioned embodiments, it will be apparent to those skilled in the art that the present invention is not limited to the embodiments described herein. The present invention can be implemented as modifications and changes without departing from the spirit and scope of the present invention defined by the description of the claims. Accordingly, the description of the present specification is for the purpose of illustration and is not intended to limit the present invention in any way.

For example, the absorbent article according to embodiments is a pants-type disposable diaper. Alternatively, the absorbent article of the present invention may be a so-called tape-type disposable diaper having a fastening tape.

### REFERENCE SYMBOLS LIST

- 10: absorbent article
- 11: absorber
- 12: waist panel
- 13: absorbent main body
- 14: front waistline region
- 15: rear waistline region
- 16: crotch region
- 18: front waistline sheet
- 19: rear waistline sheet
- 50: crotch sheet
- 51: first sheet
- 52: second sheet
- 57a: convexo-concave portions
- L: front-back direction
- W: width direction

## Claims

1. A method of manufacturing an absorbent article (10) including a waistline region (14, 15), a crotch region (16), and an absorber (11) disposed at least in the crotch region (16), the method comprising:
a shaping step (S1) of forming a convexo-concave portion (57a) on a first sheet (51) composed of a nonwoven fabric disposed in the crotch region (16) while the first sheet (51) is pulled in a conveyance direction (MD), the convexo-concave portion (57a) is convexo-concave in a thickness direction of the first sheet (51);
an absorber (11) disposing step of disposing the absorber (11) on the first sheet (51); and
an adhesion step (S8) of bonding the first sheet (51) to a waistline sheet (18, 19) disposed in the waistline region (14, 15) with a first adhesive (28, 29) after the shaping step (S1), wherein
the adhesion step (S8) includes applying at least part of the first adhesive (28, 29) to a region which overlaps with the convexo-concave portion (57a) and does not overlap with the absorber (11) in at least one of the first sheet (51) and the waistline sheet (18, 19).

2. The method of manufacturing the absorbent article according to claim 1, wherein the shaping step (S1) includes forming the convexo-concave portion (57a) extending along a direction intersecting with an orientation direction of fibers of the nonwoven fabric.

3. The method of manufacturing the absorbent article according to claim 1 or 2, wherein
the nonwoven fabric has a first surface and a second surface having a fiber density higher than a fiber density at the first surface, and wherein
the adhesion step (S8) includes bonding the second surface of the nonwoven fabric to the waistline sheet (18, 19).

4. The method of manufacturing the absorbent article according to any one of claims 1 to 3, wherein
the waistline sheet (18, 19) is composed of a nonwoven fabric,
the nonwoven fabric constituting the waistline sheet (18, 19) has a third surface and a fourth surface having a fiber density higher than a fiber density at the third surface, and
the adhesion step (S8) includes bonding the first sheet (51) to the fourth surface of the nonwoven fabric constituting the waistline sheet (18, 19).

5. The method of manufacturing the absorbent article according to any one of claims 1 to 4, wherein
the shaping step (S1) includes forming the convexo-concave portion (57a) in both end regions of the first sheet (51), the both end regions being regions of both ends of the first sheet (51) in the cross direction (CD), and
the adhesion step (S8) includes applying at least part of the first adhesive (28, 29) to the both end regions of the first sheet (51), the convexo-concave portion (57a) being formed in the both end regions, in at least one of the first sheet (51) and the waistline sheet (18, 19).

6. The method of manufacturing the absorbent article according to any one of claims 1 to 5, comprising:
a bonding step (S2) of bonding a second sheet (52) to the first sheet (51), wherein
at least one of the first sheet (51) and the second sheet (52) has a figure, a picture, or a color, and
the shaping step (S1) includes forming the convexo-concave portion (57a) in a region that does not overlap with the figure, the picture, or the color.

7. The method of manufacturing the absorbent article according to any one of claims 1 to 6, wherein the shaping step (S1) includes forming the convexo-concave portion (57a), while conveying the first sheet (51) in a conveyance direction (MD), using a roll (100) having a plurality of protrusions (102) extending in a direction (CD) intersecting the conveyance direction (MD).

8. The method of manufacturing the absorbent article according to any one of claims 1 to 6, wherein the shaping step (S1) includes forming the convexo-concave portion (57a), while conveying the first sheet (51) in a conveyance direction (MD), using a roll (200) having a plurality of protrusions (202) extending in the conveyance direction (MD).

9. The method of manufacturing the absorbent article according any one of claims 1 to 8, wherein the adhesion step (S8) includes bonding the first sheet (51) and the waistline sheet (18, 19) to each other after the first adhesive (28, 29) is applied to the waistline sheet (18, 19).

10. The method of manufacturing the absorbent article according to any one of claims 1 to 9, comprising a step (S3) of bonding an elastic member (57b) to a region of the first sheet (51), the convexo-concave portion (57a) being formed in the region, after the shaping step (S1).

11. The method of manufacturing the absorbent article according to any one of claims 1 to 10, wherein
the shaping step (S1) includes forming the convexo-concave portion (57a) in an end region of the first sheet (51) in an intersecting direction (CD) intersecting a conveyance direction (MD),
the method comprises a folding back step (S4) of folding back the first sheet (51) at an imaginary fold line (IL), as a reference point, passing through a region in which the convexo-concave portion (57a) of the first sheet (51) is formed after the shaping step (S1), and bonding the folded portion of the first sheet (51) with a second adhesive, and
the folding back step (S4) includes applying the second adhesive only to part of the folded portion.

## Patentansprüche

1. Verfahren zur Herstellung eines saugfähigen Artikels (10), der einen Taillenbereich (14, 15), einen Schrittbereich (16) und einen mindestens im Schrittbereich (16) angeordneten Absorber (11) beinhaltet, wobei das Verfahren Folgendes umfasst:
einen Formungsschritt (S1) zum Bilden eines konvex-konkaven Abschnitts (57a) auf einer ersten Schicht (51), die aus einem Vliesstoff besteht, der im Schrittbereich (16) angeordnet ist, während die erste Schicht (51) in einer Förderrichtung (MD) gezogen wird, wobei der konvex-konkave Abschnitt (57a) in einer Dickenrichtung der ersten Schicht (51) konvex-konkav ist;
einen Schritt zum Anordnen des Absorbers (11) zum Anordnen des Absorbers (11) auf der ersten Schicht (51); und
einen Klebeschritt (S8) zum Binden der ersten Schicht (51) an eine im Taillenbereich (14, 15) angeordnete Taillenschicht (18, 19) mit einem ersten Klebstoff (28, 29) nach dem Formungsschritt (S1), wobei
der Klebeschritt (S8) Auftragen mindestens eines Teils des ersten Klebstoffs (28, 29) auf einen Bereich beinhaltet, der den konvex-konkaven Abschnitt (57a) überlappt und den Absorber (11) in mindestens einer der ersten Schicht (51) und der Taillenschicht (18, 19) nicht überlappt.

2. Verfahren zur Herstellung des saugfähigen Artikels nach Anspruch 1, wobei der Formungsschritt (S1) Bilden des konvex-konkaven Abschnitts (57a) beinhaltet, der sich entlang einer Richtung erstreckt, die eine Orientierungsrichtung von Fasern des Vliesstoffs schneidet.

3. Verfahren zur Herstellung des saugfähigen Artikels nach Anspruch 1 oder 2, wobei der Vliesstoff eine erste Fläche und eine zweite Fläche aufweist, die eine Faserdichte aufweist, die höher ist als eine Faserdichte an der ersten Fläche, und wobei der Klebeschritt (S8) Binden der zweiten Fläche des Vliesstoffs an die Taillenschicht (18, 19) beinhaltet.

4. Verfahren zur Herstellung des saugfähigen Artikels nach einem der Ansprüche 1 bis 3, wobei
die Taillenschicht (18, 19) aus einem Vliesstoff besteht,
der Vliesstoff, der die Taillenschicht (18, 19) darstellt, eine dritte Fläche und eine vierte Fläche aufweist, die eine Faserdichte aufweist, die höher ist als eine Faserdichte an der dritten Fläche, und
der Klebeschritt (S8) Binden der ersten Schicht (51) an die vierte Fläche des Vliesstoffs beinhaltet, der die Taillenschicht (18, 19) darstellt.

5. Verfahren zur Herstellung des saugfähigen Artikels nach einem der Ansprüche 1 bis 4, wobei
der Formungsschritt (S1) Bilden des konvex-konkaven Abschnitts (57a) in beiden Endbereichen der ersten Schicht (51) beinhaltet, wobei die beiden Endbereiche Bereiche beider Enden der ersten Schicht (51) in der Querrichtung (CD) sind, und
der Klebeschritt (S8) Auftragen mindestens eines Teils des ersten Klebstoffs (28, 29) auf die beiden Endbereiche der ersten Schicht (51) beinhaltet, wobei der konvex-konkave Abschnitt (57a) in mindestens einer der ersten Schicht (51) und der Taillenschicht (18, 19) in den beiden Endbereichen gebildet wird.

6. Verfahren zur Herstellung des saugfähigen Artikels nach einem der Ansprüche 1 bis 5, umfassend:
einen Bindeschritt (S2) zum Binden einer zweiten Schicht (52) an die erste Schicht (51), wobei
mindestens eine der ersten Schicht (51) und der zweiten Schicht (52) eine Figur, ein Bild oder eine Farbe aufweist und
der Formungsschritt (S1) Bilden des konvex-konkaven Abschnitts (57a) in einem Bereich beinhaltet, der die Figur, das Bild oder die Farbe nicht überlappt.

7. Verfahren zur Herstellung des saugfähigen Artikels nach einem der Ansprüche 1 bis 6, wobei der Formungsschritt (S1) Bilden des konvex-konkaven Abschnitts (57a) beinhaltet, während die erste Schicht (51) in einer Förderrichtung (MD) unter Verwendung einer Walze (100) gefördert wird, die eine Vielzahl von Vorsprüngen (102) aufweist, die sich in einer Richtung (CD) erstreckt, die die Förderrichtung (MD) schneidet.

8. Verfahren zur Herstellung des saugfähigen Artikels nach einem der Ansprüche 1 bis 6, wobei der Formungsschritt (S1) Bilden des konvex-konkaven Abschnitts (57a) beinhaltet, während die erste Schicht (51) in einer Förderrichtung (MD) unter Verwendung einer Walze (200) gefördert wird, die eine Vielzahl von Vorsprüngen (202) aufweist, die sich in der Förderrichtung (MD) erstreckt.

9. Verfahren zur Herstellung des saugfähigen Artikels nach einem der Ansprüche 1 bis 8, wobei der Klebeschritt (S8) Aneinanderbinden der ersten Schicht (51) und der Taillenschicht (18, 19) beinhaltet, nachdem der erste Klebstoff (28, 29) auf die Taillenschicht (18, 19) aufgetragen worden ist.

10. Verfahren zur Herstellung des saugfähigen Artikels nach einem der Ansprüche 1 bis 9, umfassend einen Schritt (S3) zum Binden eines elastischen Elements (57b) an einen Bereich der ersten Schicht (51), wobei der konvex-konkave Abschnitt (57a) nach dem Formungsschritt (S1) in dem Bereich gebildet wird.

11. Verfahren zur Herstellung des saugfähigen Artikels nach einem der Ansprüche 1 bis 10, wobei
der Formungsschritt (S1) Bilden des konvex-konkaven Abschnitts (57a) in einem Endbereich der ersten Schicht (51) in einer Schnittrichtung (CD) beinhaltet, die eine Förderrichtung (MD) schneidet,
das Verfahren einen Rückfaltschritt (S4) zum Zurückfalten der ersten Schicht (51) an einer imaginären Faltlinie (IL) als Bezugspunkt, die durch einen Bereich verläuft, in dem der konvex-konkave Abschnitt (57a) der ersten Schicht (51) nach dem Formungsschritt (S1) gebildet wird, und Binden des gefalteten Abschnitts der ersten Schicht (51) mit einem zweiten Klebstoff umfasst und
der Rückfaltschritt (S4) Auftragen des zweiten Klebstoffs nur auf einen Teil des gefalteten Abschnitts beinhaltet.

## Revendications

1. Procédé de fabrication d'un article absorbant (10) comportant une région de ceinture (14, 15), une région d'entrejambe (16) et un absorbeur (11) disposé au moins dans la région d'entrejambe (16), le procédé comprenant :
une étape de mise en forme (S1) consistant à former une partie convexo-concave (57a) sur une première feuille (51) composée d'un non tissé disposé dans la région d'entrejambe (16) tandis que la première feuille (51) est tirée dans une direction de transport (MD), la partie convexo-concave (57a) est convexo-concave dans une direction d'épaisseur de la première feuille (51) ;
une étape de disposition de l'absorbeur (11) consistant à disposer l'absorbeur (11) sur la première feuille (51) ; et
une étape d'adhésion (S8) consistant à coller la première feuille (51) à une feuille de ceinture (18, 19) disposée dans la région de ceinture (14, 15) avec un premier adhésif (28, 29) après l'étape de mise en forme (S1), dans lequel
l'étape d'adhésion (S8) comporte l'application d'au moins une partie du premier adhésif (28, 29) sur une région qui chevauche la partie convexo-concave (57a) et ne chevauche pas l'absorbeur (11) dans au moins l'une de la première feuille (51) et de la feuille de ceinture (18, 19).

2. Procédé de fabrication de l'article absorbant selon la revendication 1, dans lequel l'étape de mise en forme (S1) comporte la formation de la partie convexo-concave (57a) s'étendant le long d'une direction croisant une direction d'orientation des fibres du non tissé.

3. Procédé de fabrication de l'article absorbant selon la revendication 1 ou 2, dans lequel le non tissé a une première surface et une deuxième surface ayant une densité de fibres supérieure à une densité de fibres au niveau de la première surface, et dans lequel
l'étape d'adhésion (S8) comporte la liaison de la seconde surface du tissu non tissé à la feuille de ceinture (18, 19).

4. Procédé de fabrication de l'article absorbant selon l'une quelconque des revendications 1 à 3, dans lequel
la feuille de ceinture (18, 19) est composée d'un non-tissé,
le non tissé constituant la feuille de ceinture (18, 19) a une troisième surface et une quatrième surface ayant une densité de fibres supérieure à une densité de fibres au niveau de la troisième surface, et
l'étape d'adhésion (S8) comporte la liaison de la première feuille (51) à la quatrième surface du non tissé constituant la feuille de ceinture (18, 19).

5. Procédé de fabrication de l'article absorbant selon l'une quelconque des revendications 1 à 4, dans lequel
l'étape de mise en forme (S1) comporte la formation de la partie convexo-concave (57a) dans les deux régions d'extrémité de la première feuille (51), les deux régions d'extrémité étant des régions des deux extrémités de la première feuille (51) dans la direction transversale (CD), et
l'étape d'adhésion (S8) comporte l'application d'au moins une partie du premier adhésif (28, 29) aux deux régions d'extrémité de la première feuille (51), la partie convexo-concave (57a) étant formée dans les deux régions d'extrémité, en au moins l'une parmi la première feuille (51) et la feuille de ceinture (18, 19).

6. Procédé de fabrication de l'article absorbant selon l'une quelconque des revendications 1 à 5, comprenant :
une étape de liaison (S2) consistant à lier une deuxième feuille (52) à la première feuille (51), dans lequel
au moins l'une parmi la première feuille (51) et la deuxième feuille (52) présente une figure, une image ou une couleur, et
l'étape de mise en forme (S1) comporte la formation de la partie convexo-concave (57a) dans une région qui ne chevauche pas la figure, l'image ou la couleur.

7. Procédé de fabrication de l'article absorbant selon l'une quelconque des revendications 1 à 6, dans lequel l'étape de mise en forme (S1) comporte la formation de la partie convexo-concave (57a), tout en transportant la première feuille (51) dans une direction de transport (MD), à l'aide d'un rouleau (100) ayant une pluralité de saillies (102) s'étendant dans une direction (CD) croisant la direction de transport (MD).

8. Procédé de fabrication de l'article absorbant selon l'une quelconque des revendications 1 à 6, dans lequel l'étape de mise en forme (S1) comporte la formation de la partie convexo-concave (57a), tout en transportant la première feuille (51) dans une direction de transport (MD), à l'aide d'un rouleau (200) ayant une pluralité de saillies (202) s'étendant dans la direction de transport (MD).

9. Procédé de fabrication de l'article absorbant selon l'une quelconque des revendications 1 à 8, dans lequel l'étape d'adhésion (S8) comporte la liaison de la première feuille (51) et de la feuille de ceinture (18, 19) l'une à l'autre après que le premier adhésif (28, 29) a été appliqué sur la feuille de ceinture (18, 19).

10. Procédé de fabrication de l'article absorbant selon l'une quelconque des revendications 1 à 9, comprenant une étape (S3) de collage d'un élément élastique (57b) à une région de la première feuille (51), la partie convexo-concave (57a) étant formée dans la région, après l'étape de mise en forme (S1).

11. Procédé de fabrication de l'article absorbant selon l'une quelconque des revendications 1 à 10, dans lequel
l'étape de mise en forme (S1) comporte la formation de la partie convexo-concave (57a) dans une région d'extrémité de la première feuille (51) dans une direction d'intersection (CD) croisant une direction de transport (MD),
le procédé comprend une étape de repliage (S4) consistant à replier la première feuille (51) au niveau d'une ligne de pliage imaginaire (IL), en tant que point de référence, passant par une région dans laquelle la partie convexo-concave (57a) de la première la feuille (51) est formée après l'étape de mise en forme (S1) et de collage de la partie pliée de la première feuille (51) avec un second adhésif, et
l'étape de repliage (S4) comporte l'application du second adhésif uniquement sur une partie de la partie repliée.
